Europäisches Patentamt

European Patent Office

Office européen des brevets

(19)

(11) Publication number : **0 523 873 A1**

(12) # EUROPEAN PATENT APPLICATION

(21) Application number : 92305925.7

(22) Date of filing : 26.06.92

(51) Int. Cl.$^5$ : **C07K 5/10, A61K 37/64**

(30) Priority : **28.06.91 US 723128**

(43) Date of publication of application :
**20.01.93 Bulletin 93/03**

(84) Designated Contracting States :
**CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Gibbs, Jackson B.**
**117 Barberry Ct.**
**Chalfont, PA 18914 (US)**
Inventor : **Garsky, Victor M.**
**754 Palmer Place**
**Blue Bell, PA 19422 (US)**
Inventor : **Rands, Elaine**
**403 Stuart Lane**
**Ambler, PA 19002 (US)**
Inventor : **Pompliano, David L.**
**1159 Gwynedale Way**
**Lansdale, PA 19446 (US)**

(74) Representative : **Thompson, John Dr. et al**
**Merck & Co., Inc. European Patent**
**Department Terlings Park Eastwick Road**
**Harlow, Essex CM20 2QR (GB)**

(54) **Non-substrate peptide inhibitors of farnesyl protein transferase.**

(57)   The present invention is directed to compounds which inhibit farnesyl-protein transferase (FTase) and the farnesylation of the oncogene protein Ras. The invention is further directed to chemotherapeutic compositions containing the compounds of this invention and methods for inhibiting farnesyl-protein transferase and the farnesylation of the oncogene protein Ras.

EP 0 523 873 A1

## BACKGROUND OF THE INVENTION

The Ras gene is found activated in many human cancers, including colorectal carcinoma, exocrine pancreatic carcinoma, and myeloid leukemias. Biological and biochemical studies of Ras action indicate that Ras functions like a G-regulatory protein, since Ras must be localized in the plasma membrane and must bind with GTP in order to transform cells. (Gibbs, J. et al., Microbiol. Rev. 53:171-286 (1989) ). Forms of Ras in cancer cells have mutations that distinguish the protein from Ras in normal cells.

At least 3 post-translational modifications are involved with Ras membrane this localization, and all 3 modifications occur at the C-terminus of Ras. The Ras C-terminus contains a sequence motif termed a "CAAX" or "Cys-Aaa$^1$-Aaa$^2$-Xaa" box (Aaa is an aliphatic amino acid, the Xaa is any amino acid) (Willumsen et al., Nature 310:583-586 (1984). Other proteins having this motif include the Ras-related GTP-binding proteins such as Rho, fungal mating factors, the nuclear lamins, and the gamma subunit of transducin.

Farnesylation of Ras by the isoprenoid farnesyl diphosphate (FPP) occurs in vivo on Cys to form a thioether linkage (Hancock et al., Cell 57:1167 (1989); Casey et al., Proc. Natl. Acad. Sci. USA 86:8323 (1989)). In addition, Ha-Ras and N-Ras are palmitoylated via formation of a thioester on a Cys residue near the C-terminal Cys farnesyl acceptor (Gutierrez et al., EMBO J. 8:1093-1098 (1989); Hancock et al., Cell 57: 1167-1177 (1989)). Ki-Ras lacks the palmitate acceptor Cys. The last 3 amino acids at the Ras C-terminal end are removed proteolytically, and methyl esterification occurs at the new C-terminus (Hancock et al., ibid). Fungal mating factor and mammalian nuclear lamins undergo identical modification steps (Anderegg et al., J. Biol. Chem 263:18236 (1988); Farnsworth et al., J. Biol Chem 264:20422 (1989)).

Inhibition of Ras farnesylation in vivo has been demonstrated with lovastatin (Merck & Co., Rahway, NJ) and compactin (Hancock et al., ibid; Casey et al., ibid; Schafer et al., Science 245:379 (1989). These drugs inhibit HMG-CoA reductase, the rate limiting enzyme for the production of polyisoprenoids and the farnesyl diphosphate precursor. It has been shown that a farnesyl-protein transferase using farnesyl-diphosphate as a precursor is responsible for Ras farnesylation (Reiss et al., Cell, 62: 81-88 (1990); Schaber et al., J. Biol Chem., 265:14701-14704 (1990): Schafer et al., Science, 249: 1133-1139 (1990): Manne et al., Proc Natl. Acad. Sci. USA, 87: 7541-7545 (1990)).

Inhibition of farnesyl-protein transferase and the farnesylation of the Ras protein blocks the ability of Ras to transform normal cells to cancer cells. The compounds of the invention inhibit Ras localization in the plasma membrane and generate soluble Ras which, as indicated infra, can act as a dominant negative inhibitor of Ras function- While soluble Ras in cancer cells can become a dominant negative inhibitor, soluble Ras in normal cells would not be an inhibitor.

A cytosol-localized (no Cys-Aaa$^1$-Aaa$^2$-Xaa box membrane domain present) and activated (impaired GTPase, staying bound to GTP) form of Ras acts as a dominant negative Ras inhibitor of membrane-bound Ras function (Gibbs et al., Proc. Natl. Acad. Sci. USA 86:6630-6634(1989)). Cytosollocalized forms of Ras with normal GTPase do not act a inhibitors. Gibbs et al., ibid, showed this effect in Xenopus oocytes and in mammalian cells.

Administration of compounds of the invention to block Ras farnesylation not only decreases the amount of Ras in the membrane but also generates a cytosolic pool of Ras. In tumor cells having activated Ras, the cytosolic pool acts as another antagonist of membrane-bound Ras function. In normal cells having normal Ras, the cytosolic pool of Ras does not act as an antagonist. In the absence of complete inhibition of farnesylation, other farnesylated proteins are able to continue with their functions.

Farnesyl-protein transferase activity may be reduced or completely inhibited by adjusting the compound dose. Reduction of farnesyl-protein transferase enzyme activity by adjusting the compound dose would be useful for avoiding possible undesirable side effects such as interference with other metabolic processes which utilize the enzyme.

These compounds and their analogs are inhibitors of farnesyl-protein transferase. Farnesyl-protein transferase utilizes farnesyl-diphosphate to covalently modify the Cys thiol group of the Ras CAAX box with a farnesyl group. Inhibition of farnesyl-diphosphate biosynthesis by inhibition of HMG-CoA reductase blocks Ras membrane localization in vivo and inhibits Ras function. Inhibition of farnesyl-protein transferase is more specific and is attended by fewer side effects than is the case of a general inhibitor of isoprene biosynthesis.

Previously, it has been demonstrated that tetrapeptide mimics of the CAAX box inhibit Ras farnesylation (Schaber et al., ibid; Reiss et al., ibid); Reiss et al., PNAS, 88: 732-736 (1991). The peptides appear to act as substrates. Since farnesylated tetrapeptides lose potency by about 15-fold (Schaber et al., ibid), it is desirable to develop tetrapeptides which act as non-substrate compounds and therefore act as true inhibitors. The novel feature of the present invention is the discovery of a class of tetrapetides that are not substrates or are poor substrates and therefore act as non-substrate inhibitors.

It is, therefore, an object of this invention to develop compounds which will inhibit farnesyl-protein transferase and the farnesylation of the oncogene protein Ras. It is a further object of this invention to develop chemotherapeutic compositions containing the compounds of this invention, and methods for producing the compounds of this invention.

## SUMMARY OF THE INVENTION

The present invention includes compounds which inhibit farnesyl-protein transferase and the farnesylation of the oncogene protein Ras, by serving as non-substrate inhibitors, chomotherapeutic compositions containing the compounds of this invention, and methods for producing the compounds of this invention.

The compounds of this invention are illustrated by the formula:

$$Cys - Xaa^1 - Xaa^2 - Xaa^3$$

wherein:

Cys is a Cysteine amino acid;

$Xaa^1$ is any amino acid;

$Xaa^2$ is the amino acid phenylalanine or a p-fluorophenylalanine; and

$Xaa^3$ is any amino acid; or the pharmaceutically acceptable salts thereof.

## DETAILED DESCRIPTION OF THE INVENTION

The compounds of this invention are useful in the inhibition of farnesyl-protein transferase and the farnesylation of the oncogene protein Ras by serving as non-substrate inhibitors. The compounds of this invention are illustrated by the formula:

$$Cys - Xaa^1 - Xaa^2 - Xaa^3$$

wherein:

Cys is a Cysteine amino acid;

$Xaa^1$ is any amino acid;

$Xaa^2$ is the amino acid phenylalanine or p-fluorophenylalanine; and

$Xaa^3$ is any amino acid; or the pharmaceutically acceptable salts thereof.

The preferred compounds of this invention include CVFM (SEQ ID NO: 1), CIFM (SEQ ID NO: 2), CI(p-fluoro-F)M and CIFQ (SEQ ID NO: 3).

In the present invention, the amino acids listed below are identified both by conventional 3 letter and single letter abbreviations as indicated below:

| | | |
|---|---|---|
| Cysteine | Cys | C |
| Glutamine | Gln | Q |
| Glycine | GLy | G |
| Isoleucine | Ile | I |
| Leucine | Leu | L |
| Lysine | Lys | K |
| Methionine | Met | M |
| Phenylalanine | Phe | F |
| Serine | Ser | S |
| Threonine | Thr | T |
| Valine | Val | V |

The pharmaceutically acceptable salts of the compounds of this invention include the conventional non-toxic salts or the quarternary ammonium salts of the compounds of this invention as formed, e.g., from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxyben-

3

zoic, fumaric, toluenesulfonic, methanesulfonic, ethane disulfonic, oxalic, isethionic, and the like.

The pharmaceutically acceptable salts of the present invention can be synthesized from the compounds of this invention which contain basic or acidic moiety by conventional chemical methods. Generally, the salts are prepared by reacting the free base or acid with stoichiometric amounts or with an excess of the desired salt-forming inorganic or organic acid or base in a suitable solvent or various combinations of solvents.

The pharmaceutically acceptable salts of the acids of the present invention are also readily prepared by conventional procedures such as treating an acid of the compounds of this invention with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine, benzylamine and the like, or a quarternary ammonium hydroxide such as tetramethylammonium hydroxide and the like.

Polyisoprenylation of Ras- [$^3$H]Farnesyldiphosphate (FPP) (20 Ci/mmol) was purchased from New England Nuclear. Farnesyl-protein transferase activity assays were carried out as 30°C unless noted otherwise. A typical reaction contained (in a final volume of 50 µL): [$^3$H]farnesyl diphosphate, Ras protein (3.5µM), 50 mM HEPES, pH 7.5, 5 mM MgCl$_2$, 5 mM dithiothreitol and farnesyl-protein transferase. After thermally pre-equilibrating the assay mixture in the absence of enzyme, reactions were initiated by the addition of farnesyl-protein transferase and stopped at timed intervals by the addition of 1 M HCl in ethanol (1 mL). The quenched reactions were allowed to stand for 15 minutes (to complete the precipitation process). After adding 2 mL of 100% ethanol, the reactions were vacuum-filtered through Whatman GF/C filters. Filters were washed four times with 2 mL aliquots of 100% ethanol, mixed with scintillation fluid (10 mL) and then counted in a Beckman LS3801 scintillation counter.

For inhibition studies, assays were run as described above, except putative inhibitors were added at the concentrations indicated. IC$_{50}$ values were determined with both farnesyl-protein transferase substrates at their K$_M$ concentrations. The K$_M$ for Ras protein substrate having the CAAX sequence, CVLS, is 3.5 µM and the K$_M$ for FPP is 0.25 µM.

When a peptide was used as the acceptor substrate, there were two changes to the assay: (1) Ras protein was excluded and, (2) the reactions were stopped by the addition of one tenth volume of 0.5 M EDTA. A portion of the reaction mixture was spotted onto a TLC plate (silica gel 60 thin layer chromatography plates (20 cm x 20 cm, 0.25 mm layer thickness) were from E. Merck (Darrnstadt)) and eluted with either ethyl acetate:pyridine:acetic acid:water (10:5:1:3) or n-propanol:water (7:3) elution systems. After drying under vacuum, the plates were sprayed with En$^3$Hance and exposed to X-ray film at -70°C.

Ras proteins used as substrates were expressed in E. coli, and purified (Gibbs et al. Proc. Natl. Acad. Sci. USA 85:5026-5030(1988)). Farnesyl-protein transferase was prepared from bovine brain. All purification steps were performed at 4°C. Cerebral lobes from bovine brains were homogenized in lysis buffer containing 50 mM. Tris-Cl, pH 8.0, 1 mM EGTA, 1 mM MgCl$_2$, 5 mM dithiothreitol, 10 µg/mL aprotinin, 0.5 mM phenylmethyl sulfonyl fluoride (PMSF), 2 µg/mL anitpain and 2 µg/mL leupeptin. Cellular debris and membranes were removed by centrifugation (10,000 g for 20 minutes followed by 100,000 g for 30 minutes). The supernatant was loaded directly onto a column (30 cm x 20 cm$^2$) of DEAE-Sephacel that had been equilibrated with lysis buffer. The column was washed with the same buffer and proteins were eluted with a linear gradient of NaCL (0 - 500 mM, 1 L + 1 L) in the same buffer. Fractions (20 mL) were collected and those containing farnesyl-protein transferase activity were pooled. Each pool was then applied to an ω-Aminooctyl agarose column (30 cm x 4.9 cm$^2$) and eluted with a linear gradient of NaCl (0 - 500 mM, 500 mL + 500 mL) in lysis buffer. Fractions containing farnesylprotein transferase were pooled. For most assays further partial purifiation was achieved by HPLC using a Mono Q H.R. 10/10 column and eluting farnesyl-protein transferase activity with a 0-0.3 M NaCl gradient over 85 minutes at 2 ml/min.

To prepare pure enzyme, the nonpeptide, KSLTGCVIM, was covalently bound to Affigel-10 under anhydrous conditions as described by the manufacture (15 µmol peptide per 1 mL of Affigel-10). Unreacted resin was treated with ethanolamine. After multiple passes of ω-aminooctyl-agarose-column-purified protein over the peptide column, the column was washed and eluted as described by Reiss, et al., (1990) ibid, to recover farnesyl-protein transferase activity.

The compounds of the invention can be synthesized from their constituent amino acids by conventional peptide synthesis techniques, preferably by solid-phase technology. The peptides are then purified by reverse-phase high performance liquid chromatography (HPLC).

Standard methods of peptide synthesis are disclosed, for example, in the following works: Schroeder et al., "The Peptides", Vol. I, Academic Press 1965, or Bodanszky et al., "Peptide Synthesis", Interscience Publishers, 1966, or McOmie (ed.) "Protective Groups in Organic Chemistry", Plenum Press, 1973, or Barany et al., "The Peptides: Analysis, Synthesis, Biology" 2, Chapter 1, Academic Press, 1980. The teachings of these works are hereby incorporated by reference.

Peptides were prepared with an Applied Biosystems model 430A peptide synthesizer, purified by reverse-phase HPLC, and characterized by amino acid analysis and fast atom bombardment mass spectrometry. Chemical farnesylation was achieved by reacting trans, trans-farnesyl bromide (Aldrich) with unprotected peptide.

Farnesylation of Ras in vitro- To evaluate in vitro polyisoprenylation of Ras, we have used two engineered S. cerevisiae RAS gene products were used, (Leu[68]RASI(term.) and (Leu[68]RASI(term.)CVLS (CVLS=Cys-Val-Leu-Ser) described by Gibbs et al., Proc. Natl. Acad. Sci. USA 86:6630-6634. Both proteins contain the first 184 amino acids of yeast RASI which do not have any Cys residues. [Leu[68]]RASI(term.) ends with a Pro substitution for Leu-185. (Leu[68]RASI (term.)CVLS has the final Pro residue replaced by an additional seven C-terminal residues having the sequence -Ser-Leu-Lys-Cys-Val-Leu-Ser, wherein this protein is herein called 8as-CVLS. Purified Ras protein (3.5 $\mu$M) was incubated with partially pure enzyme in the presence of 0.25 $\mu$M [$^3$H] FPP. Radioactive precursor was incorporated into Ras-CVLS. No detectable labeling was observed in reactions lacking Ras or having [Leu[68]]RASI-(term.) suggesting that modification was occurring at the unique C-terminal region of Ras-CVLS. Ras-CVLS was positively identified as the acceptor protein by immunoprecipitation with the Ras-specific monoclonal antibody Y13-259. Both normal and oncogenic mammalian Ha Ras proteins also served as substrates.

CAAX tetrapeptides and other tetrapeptides including Cys-Xaa[1]-Xaa[2]-Xaa[3] were tested as farnesylation substrates using [$^3$H]FPP and partially pure bovine brain farnesyl-protein transferase. Our earlier findings with the heptapeptide SSGCVLS suggested that it served as a substrate for isoprenylation (Schaber et al., ibid). Thin layer chromatography (TLC) conditions were developed which resolved the substrates farnesyl diphosphate ($R_f$ = 0.06) and CVLS ($R_f$ = 0.28) and product S-farnesyl-CVLS ($R_f$ = 0.58). These $R_f$ values reflect TLC conditions using ethylacetate: pyridine:acetic acid:water (10:5:1:3) as the elutent. In another elution system, n-propanol: water (7:3), farnesyl-diphosphate ($R_f$ = 0.18) was readily separated from farnesylated tetrapeptides (examples: CAIS ($R_f$ = 0.84) and CAIM ($R_f$ = 0.83). Quenched reaction mixtures containing peptide, farnesyl-diphosphate and farnesyl-protein transferase, were separated by TLC followed by visualization of the radioactive species by autoradiography. In the control reaction (no peptide), only unreacted starting material and farnesyl-diphosphate could be seen, indicating that the partially purified enzyme preparation had little phosphatase activity. In the presence of the peptide CVLS, a radioactive spot was observed that co-migrated with the S-farnesyl-CVSL standard. Control peptides having a Cys to Ser substitution were not substrates.

The results of these experiments showing activity are shown in Table 1. The criticality of Cys-Xaa[1]-Xaa[2]-Xaa[3] as defined in this pecification is shown.

EP 0 523 873 A1

## Table 1

| Peptide (200µM) | Farnesylation Substrate |
|---|---|
| CVLS | + |
| SVLS | − |
| CAIS | + |
| CAIM | + |
| CYIM | + |
| CVIM | + |
| CVFM (SEQ ID NO: 1) | − |
| CIFM (SEQ ID NO: 2) | − |
| CIFQ (SEQ ID NO: 3) | − |
| CI(p-fluoro-F)M | − |

+ 〉 90% [3H] farnesyl diphosphate converted to [3H] farnesyl tetrapeptide

− 〈 1% conversion

The indicated peptides at 200 µM were incubated with partially pure bovine farnesyl-protein transferase and partially pure bovine farnesyl-protein transferase and 0.5 µM [3H]-FPP for 30 minutes at 30°C. Autoradiographic exposure time was overnight (15-20 hours).

To determine whether tetrapeptides that were not substrates could bind to farnesyl-protein transferase, they were tested for the ability to inhibit Ras farnesylation in vitro(Table 2).

## Table 2. Inhibition of Ras farnesylation by tetrapeptides.

| Compound | $IC_{50}$ (nM) |
|---|---|
| CVLS | 2000 |
| CAIS | 1000 |
| CAIM | 200 |
| CYIM | 100 |
| CVIM | 100 |
| CVFM (SEQ ID NO: 1) | 20 |
| CIFM (SEQ ID NO: 2) | 10 |
| CIFQ (SEQ ID NO: 3) | 23 |
| CI(p-fluoro-F)M | 10 |

Farnesyl-protein transferase from bovine brain was chromatographed on DEAE-Sephacel (Pharmacia, 0-0.8 M NaCl gradient elution), N-octyl agarose (Sigma, 0-0.6 M NaCl gradient elution), and a Mono Q HPLC column (Pharmacia, 0.0.3M NaCl gradient). Ras-CVLS at 3.5 µM, 0.25 µM [3H]FPP, and the indicated compounds were

6

incubated with this partially purified enzyme preparation. Data presented in the Table 2 are the average of 2-5 determinations. Single capital letter amino acid abbreviations are used above.

The inhibition data indicates that Cys-Xaa$^1$-Xaa$^2$-Xaa$^3$ tetrapeptides of the invention bind to farnesyl-protein transferase. To further verify that Cys-Xaa$^1$-Xaa$^2$-Xaa$^3$ were interacting with the active site of farnesyl-protein transfer, CIFM (SEQ ID NO: 2) was evaluated for its mechanism of inhibition using homogenous, pure farnesyl-protein transferase. CIFM (SEQ ID NO: 2) was observed to act as a competitive inhibitor with respect to Ras protein substrate.

CIFM (SEQ ID NO: 2) was not a competitive inhibitor with respect to farnesyl diphosphate. Thus, CIFM (SEQ ID NO: 2) binds to the protein substrate binding site of farnesyl-protein transferase, but CIFM (SEQ ID NO: 2) and tetrapeptides of the invention Cys-Xaa$^1$-Xaa$^2$-Xaa$^3$ do not serve as effective substrates in the reaction.

The compounds of this invention inhibit farnesyl-protein transferase and the farnesylation of the oncogene protein Ras. These compounds are useful as pharmaceutical agents for mammals, especially for humans. These compounds may be administered to patients for use in the treatment of cancer. Examples of the type of cancer which may be treated with the compounds of this invention include, but are not limited to, colorectal carcinoma, exocrine pancreatic carcinoma, and myeloid leukemias.

The compounds of this invention may be administered to mammals, preferably humans, either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intreperitoneal, substaneous and topical administration.

For oral use of a chemotherapeutic compound according to this invention, the selected compounds may be administered, for example, in the form of tablets or capsules, or as an aqueous solution or suspension. In the case of tablets for oral use, carriers which are commonly used include lactose and corn starch, and lubricating agents such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramusclar, intraperitioeal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

The present invention also encompasses a pharmaceutical compostion useful in the treatment of cancer, comprising the administration of a therapeutically effective amount of the compounds of this invention, with or without pharmaceutically acceptable carriers, e.g. saline, at a pH level e.g. 7.4. The solutions may be introduced into a patients intramuscular blood-stream by local bolus injection.

When a compound according this invention is administered into a human subject, the daily dosage will normally be determined by the prescribing physician with the dosage generally varying according to the age, weight, and response of the individual patient, as well as the severity of the patient's symptoms.

In one examplary application, a suitable amount of compound is administered to a patient undergoing treatment for cancer. Administration occurs in an amount between about .1 mg/kg of body weight to about 20 mg/kg of body weight of a mammal per day, preferably of between .5 mg/kg of body weight to about 10 mg/kg of body weight of a mammal per day.

## EXAMPLES

Examples provided are intended to assist in a further understanding of the invention. Particular materials employed species and conditions are intended to be further illustrative of the invention and not limitative of the reasonable scope thereof.

## EXAMPLE 1

### Preparation of CVFM (SEQ ID NO: 1)

Starting with 0.5 moles of t-Boc-L-methionine (phenylacetamidomethyl resin) (which is commercially available) the following amino acids were introduced using an automated Applied Biosystems peptide synthesizer: t-Boc-L-phenylalanine, t-Boc-L-valine, t-Boc-S-4-methylbenzyl-L-cysteine. Couplings were mediated by dicyclohexylcarbodiimide to introduce each amino acid in its symmetrical anhydride form. Deblocking of the t-Boc group (tert-butyloxycarbonyl) was achieved with TFA (trifluoroacetic acid). At the completion of the assembly of the protected peptide intermediate, t-Boc-S-4-methylbenzyl-L-cysteinyl-L-valyl-L-phenylalanyl-L-methionyl

phenylacetamidomethyl resin, the t-Boc protecting group was removed with TFA and the resin bound peptide dried. The peptide was cleaved from the resin using liquid HF (hydrogen fluoride) in the presence of anisole as a scavenger at 0°C for 1 hr. Following evaporation, the residual mixture was ether washed, filtered and the peptide extracted away from the resin with $H_2O$. The aqueous solution of crude product was freeze-dried. Purification of the peptide, Cys-Val-Phe-Met, (CVFM) (SEQ ID NO: 1) was performed by reverse phase liquid chromatography on a C-18 silica based support. The gradient elution of the desired product was achieved using 0.1% aqueous TFA and 0.1% TFA in acetonitrile as buffers for elution. The purified product fractions were pooled and freeze dried. Identity and homogeneity were established by amino acid composition, analytical HPLC and mass spectroscopy.

EXAMPLE 2

Preparation of CIFM (SEQ ID NO: 2)

Starting with 0.5 mmoles of t-Boc-L-methionine (phenylacetamidomethyl resin) (which is commercially available) the following amino acids were introduced using an automated Applied Biosystems peptide synthetizer: t-Boc-L-phenylanaline, t-Boc-L-isoleucine, t-Boc-S-4-methylbenzyl-L-cysteine. Couplings were mediated by dicyclohexylcarboxdiimide to introduce each amino acid in its symmetrical anhydride form. Deblocking of the t-Boc group (tert-butyloxycarbonyl) was achieved with TFA (trifluoroacetic acid). At a completion of the assembly of the protected peptide intermediate, t-Boc-S-4-methylbenzyl-1-cysteinyl-L-isoleucyl-L-phenylalanyl-L-methionyl-phenylacetamidomethyl resin, the t-Boc-protecting group was removed with TFA and the resin bound peptide dried. The peptide was cleaved from the resin using liquid HF (hydrogen fluoride) in the presence of anisole as a scavenger at 0°C for 1 hr. Following evaporation, the residual mixture was ether washed, filtered and the peptide extracted away from the resin with $H_2O$. The aqueous solution of crude product was freeze-dried. Purification of the peptides, Cys-Ile-Phe-Met, (CIFM) (SEQ ID NO: 2) was performed by reverse phase liquid chromatography on a C-18 silice based support. The gradient elution of the desired product was achieved using 0.1% aqueous TFA and 0.1% TFA in acetonitrile as buffers for elution. The purified product fractions were pooled and freeze-dried. Identity and homogeneity were established by amino acid composition, analytical HPLC and mass spectroscopy.

EXAMPLE 3

Preparation of CIFQ (SEQ ID NO: 3)

Starting with 0.5 mmoles of t-Boc-L-glutamine (phenylacetamidomethyl resin) (which is commercially available) the following amino acids were introduced using an automated Applied Biosystems peptide synthesizer: t-Boc-L-phenylalanine, t-Boc-L-isoleucine, t-Boc-S-4- methylbenzyl-L-cysteine. Couplings were mediated by dicyclohexylcarbodiimide to introduce each amino acid in its symmetrical anhydride form. Deblocking of the t-Boc group (tert-butyloxy-carbonyl) was achieved with TFA (trifluoroacetic acid). At the completion of the assembly of the protected peptide intermediate, t-Boc-S-4-methylbenzyl-L-cysteinyl-L-isoleucyl-L-phenylalanyl-L-glutaminyl-phenyl-acetamidomethyl resin, the t-Boc protecting group was removed with TFA and the resin bound peptide dried. The peptide was cleaved from he resin using liquid HF (hydrogen fluoride) in the presence of anisole as a scavenger at 0°C for 1 hr. Following evaporation, the residual mixture was ether washed, filtered and the peptide extracted away from the resin with $H_2O$. The aqueous solution of crude product was freeze-dried. Purification of the peptide, Cys-Ile-Phe-Gln, (CIFQ) (SEQ ID NO: 3) was performed by reverse phase liquid chromatography on a C-18 silica based support. The gradient elution of he desired product was achieved using 0.1% aqueous TFA and 0.1% TFA in acetonitrile as buffers for elution. The purified product fractions were pooled and freeze-dried. Identity and homogeneity were established by amino acid composition, analytical HPLC and mass spectroscopy.

## Claims

1. A compound which inhibits farnesyl-protein transferase and prevents transformation of normal cells into cancer cells by acting as a non-substrate inhibitor, which compound comprises the amino acid sequence:

$$Cys - Xaa^1 - Xaa^2 - Xaa^3$$

wherein:

Cys is a Cysteine amino acid;
Xaa$^1$ is any amino acid;
Xaa$^2$ is the amino acid phenylalanine or p-fluorophenylalanine; and
Xaa$^3$ is any amino acid; or the pharmaceutically acceptable salts thereof.

2. A compound according to Claim 1 which is CVFM (SEQ ID NO: 1).

3. A compound according to Claim 1 which is CIFM (SEQ ID NO: 2).

4. A compound according to Claim 1 which is CIFQ (SEQ ID NO: 3).

5. A compound according to Claim 1 which is CI(p-fluoro-F)M.

6. A chemotherapeutic composition comprising a pharmaceutical carrier, and dispersed therein a therapeutically effective amount of a compound according to any one of the preceding Claims.

7. The use of a composition of Claim 6 for the manufacture of a medicament for inhibiting farnesylprotein transferase and plasma membrane Ras protein localization, and preventing transformation of normal cells into cancer cells by acting as a non-substrate inhibitor.

8. The use of a composition of Claim 6 for the manufacture of a medicament for treating cancer.

9. The use according to Claim 7 or Claim 8, wherein the mammal is a human.

European Patent
Office
**EUROPEAN SEARCH REPORT**
Application Number

EP    92 30 5925
Page  1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROCEEDINGS OF THE NATIONAL ACADEMY OF SCIENCES USA<br>vol. 88, February 1991, WASHINGTON, USA<br>pages 732 - 736<br>REISS ET AL 'Sequence requirement for peptide recognition by rat brain p21-ras protein farnesyltransferase'<br>* See page 732, Abbreviation *<br>* See page 734, Figure 4 *<br>--- | 1-2 | C07K5/10<br>A61K37/64 |
| X,O | EDITORS: SMITH ET AL // AUTHORS: STRADLEY ET AL 'Proc. 12th Am. Pept. Symp.,June 16-21 1991, USA //  Tetrapeptides compete with p21-ras for farnesylation catalyzed by protein farnesyltransferase'<br>1992 , ESCOM , LEIDEN, HOLLAND<br>* See page 934, Table 1 *<br>--- | 1-2 | |
| A | PROCEEDINGS OF THE NATIONAL ACADEMY OF SCIENCES USA<br>vol. 85, July 1988, WASHINGTON, USA<br>pages 4643 - 4647<br>CLARKE ET AL 'Posttranslational modification of the Ha-ras oncogene protein: Evidence for a third class of protein carboxyl methyltransferas'<br>* See page 4644, Table 1 *<br>--- | 1-9 | TECHNICAL FIELDS SEARCHED (Int. Cl.5)<br><br>C07K |
| A | JOURNAL OF THE AMERICAN CHEMICAL SOCIETY<br>vol. 91, no. 13, 1969, WASHINGTON, USA<br>pages 3426 - 3428<br>HAVRAN ET AL 'The synthesis and pharmacological properties of [2-isoleucine]-8-lysine-vasopressin and its 1-deamino analog'<br>* See page 3627, column 2, bottom *<br>--- | 4 | |

-/--

The present search report has been drawn up for all claims

| Place of search | Date of completion of the search | Examiner |
|---|---|---|
| THE HAGUE | 11 NOVEMBER 1992 | KORSNER S.E. |

**European Patent Office**

## EUROPEAN SEARCH REPORT

Application Number

EP 92 30 5925
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X,P | JOURNAL OF BIOLOGICAL CHEMISTRY vol. 266, no. 24, August 1991, BALTIMORE, USA pages 15575 - 15578 GOLDSTEIN ET AL 'Nonfarnesylated tetrapeptide inhibitors of protein farnesyltransferase' * See page 15575, Table 1 * | 1-3 | |
| X,P | WO-A-9 116 340 (BOARD OF REGENTS, THE UNIVERSITY OF TEXAS SYSTEM) 31 October 1991 | 1-2 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 11 NOVEMBER 1992 | KORSNER S.E. |

SEQUENCE LISTING

(1) GENERAL INFORMATION:

    (i) APPLICANT: Gibbs, Jackson B
                  Garsky, Victor M
                  Rands, Elaine R
                  Pompliano, David L

    (ii) TITLE OF INVENTION: Non-Substrate Inhibitors of Farnesyl
          Protein Transferase

    (iii) NUMBER OF SEQUENCES: 3

    (iv) CORRESPONDENCE ADDRESS:
        (A) ADDRESSEE: Merck & Co., Inc.
        (B) STREET: P.O. Box 2000
        (C) CITY: Rahway
        (D) STATE: New Jersey
        (E) COUNTRY: USA
        (F) ZIP: 07065-0900

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER: US 723,128
        (B) FILING DATE: 28-JUN-1991
        (C) CLASSIFICATION: Original

    (viii) ATTORNEY/AGENT INFORMATION:
        (A) NAME: Daniel, Mark R
        (B) REGISTRATION NUMBER: 31,913
        (C) REFERENCE/DOCKET NUMBER: 18456

    (ix) TELECOMMUNICATION INFORMATION:
        (A) TELEPHONE: (908) 594-6609
        (B) TELEFAX: (908) 594-4720

```
(2) INFORMATION FOR SEQ ID NO:1:

    (i) SEQUENCE CHARACTERISTICS:
        (A) LENGTH: 4 amino acids
        (B) TYPE: amino acid
        (C) STRANDEDNESS: unknown
        (D) TOPOLOGY: linear

    (ii) MOLECULE TYPE: peptide



    (xi) SEQUENCE DESCRIPTION: SEQ ID NO:1:

    Cys Val Phe Met
    1

(2) INFORMATION FOR SEQ ID NO:2:

    (i) SEQUENCE CHARACTERISTICS:
        (A) LENGTH: 4 amino acids
        (B) TYPE: amino acid
        (C) STRANDEDNESS: unknown
        (D) TOPOLOGY: linear

    (ii) MOLECULE TYPE: peptide



    (xi) SEQUENCE DESCRIPTION: SEQ ID NO:2:

    Cys Ile Phe Met
    1

(2) INFORMATION FOR SEQ ID NO:3:

    (i) SEQUENCE CHARACTERISTICS:
        (A) LENGTH: 4 amino acids
        (B) TYPE: amino acid
        (C) STRANDEDNESS: unknown
        (D) TOPOLOGY: linear

    (ii) MOLECULE TYPE: peptide



    (xi) SEQUENCE DESCRIPTION: SEQ ID NO:3:

    Cys Ile Phe Gln
    1
```